Europäisches Patentamt

European Patent Office

Office européen des brevets

(11) Publication number: **0 179 576**
A2

## (12) EUROPEAN PATENT APPLICATION

(21) Application number: 85306855.9

(22) Date of filing: 26.09.85

(51) Int. Cl.⁴: **C 12 P 21/00**
**A 61 K 39/395**

(30) Priority: 19.10.84 JP 218678/84

(43) Date of publication of application:
30.04.86 Bulletin 86/18

(84) Designated Contracting States:
AT BE CH DE FR GB IT LI LU NL SE

(71) Applicant: TAISHO PHARMACEUTICAL CO. LTD
24-1 Takata 3-chome Toshima-ku
Tokyo 171(JP)

(72) Inventor: Ohno, Tsuneya
5-15, Fukazawa 2-chome
Setagaya-ku Tokyo(JP)

(74) Representative: Myerscough, Philip Boyd et al,
J.A.Kemp & Co. 14, South Square Gray's Inn
London, WC1R 5EU(GB)

(54) Monoclonal antibody.

(57) A hybridoma cell which produces a monoclonal antibody having specificity to immunologically tolerant hormone or enzyme is efficiently isolated by immunizing an animal, such as a mouse, which is in a state of autoimmune diseases or hereditarily susceptible to autoimmune diseases with an immunologically tolerant hormone (such as insulin) or enzyme (such as aequorin) as an antigen, fusing antibody-producing cells from the immunized animal with myeloma cells such as mouse myeloma cells to form hybridoma cells and cloning the hybridoma cells capable of producing a monoclonal antibody having specificity to the hormone or enzyme.

EP 0 179 576 A2

## MONOCLONAL ANTIBODY

1.  Field of the Invention

This invention relates to a monoclonal antibody to a hormone or enzyme which is immunologically tolerant to an animal to be immunized, and a process for producing cells capable of forming the monoclonal antibody.

2.  Description of the Prior Art

A process for producing a specific antibody by using hybridoma cells obtained by the fusion of spleen cells with myeloma cells was reported by Koehler and Milstein [G. Koehler and G. Milstein, Nature, 256, 495 (1975)], and various improvements have been made on it [G. Galfre, S. C. Howe, C. Milstein, G. W. Butcher and J. C. Howard, Nature, 266, 550 (1977); G. Galfre, C. Milstein and B. Wright, Nature, 277, 131 (1979); and M. Shulman and C. Koehler, Nature, 276, 269 (1978)]. It is known however that an animal organism generally cannot produce an antibody to a substance which is immunologically tolerant to itself, namely a substance which is recognized as "self" by the immunocompetent cells of that organism. The known processes cannot produce, or can produce only in very low ratios, hybridoma cells which form antibodies having specificity to biologically active substances or other special substances, such as hormones or enzymes.

The present inventor and his co-workers reported in Proceedings of the Japanese Cancer Association, The 43d Annual Meeting, Ocober 1984 (Fukuoka, Japan) that a monoclonal antibody to gp52 of envelope proteins of mouse mammary tumor virus (MMTV) can be produced in high ratios by using a mouse (MRL/1) being hereditarily susceptible to autoimmune disease or being in a state of autoimmune disease as an animal to be immunized.

SUMMARY OF THE INVENTION

The present inventor turned his attention to the use of an animal which is in a state of autoimmune diesease or hereditarily susceptible to autoimmune disease as an animal to be immunized, and has now succeeded in establishing a technique of producing hybridoma cells capable of producing a monoclonal antibody to a hormone or enzyme by using that animal.

The monoclonal antibody produced by the process of this invention is a novel and very useful substance not described in the prior literature. For example, the use of this monoclonal antibody can lead to the establishment of a method of ultra microdetermination of a biologically active substance such as a hormone or enzyme to the order of micrograms to nonagrams.

Insulin is a substance necessary for the treatment of diabetes. Insulin preparations now in therapeutic use are obtained by humanizing porcine-derived insulin by changing its amino acid sequence. Clinical cases have been reported however in which on long-term administration of the humanized porcine-derived insulin to a human patient, the patient's body produces an antibody to that insulin unlike the case of administering insulin purely derived from humans, and treatment with the insulin can not be continued. It is not clear whether this is because the humanized porcine-derived insulin is harmful or it is attributed to traces of impurities contained in the insulin preparations. By applying the process of this invention, however, various anti-insulin antibodies can be obtained, and the use of these anti-insulin antibodies will lead to the elucidation of a cause for the above cases. Furthermore, by using an affinity column prepared from such an anti-insulin antibody, it will be possible to develop a method of efficiently removing the aforesaid impurities from the insulin preparations. The above mentioned methods can be also applied to insulins produced by the technique of the genetic engineering.

This invention can give monoclonal antibodies specific to various immunologically tolerant substances. The use of the specific monoclonal antibodies enables the existence in vivo of the immunologically tolerant substances to be detected, and thus serves for the diagnosis of various diseases. Furthermore, by means of affinity columns prepared by using these specific monoclonal antibodies, traces of immunologically tolerant biologically active substances can be purified in large quantities, and the purified substances can be utilized for the treatment and therapy of various diseases which are now difficult to cure.

The present invention provides a monoclonal antibody having specificity to an immunologically tolerant hormone or enzyme and a process for producing cells capable of producing the monoclonal antibody. The antibody is produced from hybridoma cells prepared by a method which comprises immunizing an animal being in a state of autoimmune disease or herediterily susceptible to autoimmune disease with a hormone or enzyme which is immunologically tolerant to a normal animal of the same species as said anumal, fusing the resulting antibody-producing cells wth myeloma cells, isolating the antibody-producing hybridoma cells thus obtained, and cloning the above hybridoma cells.

DETAILED DESCRIPTION OF THE INVENTION

The term "autoimmune disease", as used in this invention, denotes a diseased condition of an animal organism in which the organism's recognition mechanism of distinguishing between "self" and "nonself" is imperfect, and the organism produces an antibody also to a substance which is intrinsically recognized as "self" by the immunocompetent cells of that organism and to which no antibody is intrinsically produced. For detailed information on autoimmune diseases, see, for example, THEOFILOPOULOS, A., DIXON, F. (1982), Am. J. Pathol. 108, 321-365.

The animal "being hereditarily susceptible to autoimmune disease", as used herein, denotes experimental animals which are inbred as an inbred strain (a strain hereditarily purified by sequential brother-sister matings), and in which at least 90% of the individuals fall into autoimmune disease.

In the technique of producing a monoclonal antibody, it is first necessary to immunize an animal with an antigen. Mice, particularly BALB/c strain mice, are used as the animal to be immunized because it is generally believed that the use of spleen cells from a mouse of the same strain and species (BALB/c) as a mouse from which the myeloma cells are derived is desirable for performing cell fusion. The present inventor, tried, but almost failed, to obtain hybridoma cells capable of producing an antibody specific to a certain immunologically tolerant substance when he used BALB/c strain mice.

It has now been found in accordance with this invention that hybridoma cells capable of producing the desired antibody in a very high ratio can be prepared by deriving immunized spleen cells from a $F_1$ hybrid of a NZB strain mouse and a NZW strain mouse, (NZBxNZW) $F_1$, or a mouse of the NZB, BXSB, MRL/l or MRl/n strain, which is hereditarily liable to fall into autoimmunity (such mice will be referred to hereinafter as "autoimmune mice"), and fusing the spleen cells with myeloma cells.

It is known that these autoimmune mice are of an experimental animal strain which assumes a condition very close to SLE (systemic lupus erythematosus), one human autoimmune disease. In the present invention, mice 1 to 2 weeks before their spontaneous onset of the disease are used. The autoimmune mice are immunized with an immunologically tolerant substance. The immunization is repeated until the antibody titer becomes sufficiently high. Three days after the final immunization, antibody-producing cells, for example spleen cells, are extracted from the immunized animals, and fused with myeloma cells by a cell fusion technique to produce hybridomas of the spleen cells and myeloma cells which produce an antibody specific to the immunologically tolerant substance. The fusion of the spleen cells with the myeloma cells can be easily carried out by a cell fusion method known per se, for example the method reported by Koehler and Milstein mentioned above. The hybridoma cells can be obtained in high yields by using a limited selective medium in which only the fused cells (hybridoma cells) survive, such as HAT selective medium.

The term "immunologically tolerant substance", as used in the present application, denotes a substance which is recognized as "self" by the immunocompetent cells of an animal organism being in normal condition, and therefore to which no antibody is produced by that organism. The immunocompetent cells of the normal organism recognize all its constituent elements and products as "self", and produce no antibody to these substances. Examples of such substances are hormones, neurotransmitter factors and enzymes produced in vivo.

A substance having the same activity but quite different structures in different biological species, for example a human and a mouse, is an exception and an antibody to it is formed in a different species. Generally, however, a hormone or enzyme has very little structural difference between different biological species, and therefore, it is frequently very difficult to produce an antibody to such a substance even in a different species.

The hybridoma cells prepared as above are then screened to select those which have the ability to produce an antibody having specificity to the antigen used in the immunization (the immunologically tolerant substance). The selected hybridoma cells are then cloned. The screening and cloning can be carried out by methods known per se (see, for example, the aforesaid publication by Koehler and Milstein). For example, by screening the hybridoma cells by enzyme-linked immunosorbent assay (ELISA), and cloning the selected hybridoma cells by the limited dilution method, a hybridoma cell line capable of producing a monoclonal antibody to the antigen can be obtained.

The cell fusion technique enables a homogeneous monoclonal antibody to be produced continuously by cultivating the fused cells. A general outline of this production method has already been known (see, for example, the publication by Koehler and Milstein), and the present invention can utilize it directly. The aforesaid publication of Koehler and Milstein is therefore cited herein in lieu of a detailed description of the cell fusion technique applicable to the present invention.

The process of this invention described above demonstrates that by immunizing an autoimmune animal with an immunologically tolerant substance, its immunological tolerance can be easily destroyed. According to the process of this invention, an antibody-producing cell line can be established to an immunologically tolerant substance, such as a hormone or enzyme to which an antibody has been considered to be difficult to produce by conventional methods.

The resulting antibody-producing cell line can be propagated _in vitro_ or in the abdominal cavity of an animal, and a monoclonal antibody can be recovered from the culture fluid or ascites. For example, all hybridoma cells obtained by fusion of spleen cells of NZBXNZW, $F_1$ mice or MRL/1 strain mice with mouse myeloma cells (NS-1) can propagate in the abdominal cavity of BALB/c strain mice, and the monoclonal antibodies are secreted in the ascites of the host mice.

The _in vitro_ propagation can be carried out, for example, in a replicating medium such as a complete medium [RPMI-1640 supplemented by 2 mM glutamine, 1mM sodium pyruvate, fungizone (0.25 microgram/ml) Streptomycin (150 ng/ml), penicillin (50 units/ml) and 15% bovine serum (screened)] at 37$^O$C in the presence of 5% $CO_2$ in an incubator. The hybridoma cells ($2 \times 10^6$ to $1 \times 10^7$) so

cultivated may be inoculated in the abdominal cavity of a mouse. Desirably, the mouse used for inoculation is pretreated by intraperitoneally injecting 0.5 ml of pristane (a product of Pfalts & Bauer Company) 1 to 2 weeks before the inoculation. Generally, the ascites can be extracted 4 to 10 days after the inoculation. The ascites contain the hybridoma cells and antibodies produced by the hybridoma cells. The hybridoma cells are removed by centrifugation, and the supernatant is used as an antibody fluid.

The following Examples illustrate the process of this invention further.

### EXAMPLE 1
Production of cells capable of producing
a monoclonal antibody to aequorin:-

Aequorin is a special photoprotein isolated from a jellyfish (Aequorea) and is known to emit unique fluorescence when combined with $Ca^{++}$ ion molecules [F. Johnson and O. Shimomura: Nature, New Biology, 237, 287-288, (1972)]. By utilizing this property, it can be used for elucidating muscular contraction, for example for determining $Ca^{++}$ in cells. An antibody specific to it gives us effective means for such studies.

NZBxNZW, $F_1$ mice were immunized with purified aequorin, and their ability to produce an antibody was examined. Comparative experiments were conducted by using NZBxHZW, $F_1$ mice of different ages and also administering the same antigen to BALB/c strain mice used generally for production of monoclonal antibodies.

Specifically, the antigen aequorin was injected into three groups of mice in an amount of 20 micrograms for the first time, 10 micrograms for the second time and 5 micrograms finally. The three groups were a group of NZBxNZW, $F_1$ mice (5 weeks old), a group of mice of the same strain (11 weeks old) and a group of BALB/c mice (5 weeks old).

0179576

After the immunization, spleen cells were extracted from the immunized animals, and fused with mouse myeloma cells (NS-1) by the PEG method, and the resulting hybridoma cells were propagated in HAT selective medium. Consequently, as shown in Table 1, 116 hybridomas were obtained from the group of NZBxNZW, $F_1$ mice with an age of 5 weeks, 27 hybridomas from the group of NZBxNZW, $F_1$ mice with an age of 11 weeks, and as high as 662 hybridomas from the group of BALB/c mice. In the case of the BALB/c mouse group, however, antibodies to aequorin were produced from 5 hybridomas and the ratio of antibody formation was only 0.7%. In contrast, 22 hybridomas obtained from the NZBxNZW, $F_1$ mice with an age of 5 weeks produced specific antibodies, and the ratio of the specific antibodies formed was as high as 19.0%. With the 11 week-old mice, both the ratio of hybridoma formation and the ratio of antibody production (18.5%) tended to decrease, as shown in Table 1. These results suggest the effect of the increased age.

For details of the PEG method used, see G. Galfre, S. C. Howe, C. Milstein, G. W. Butcher and J. C. Howard: Nature, 266, 550 (1977). The HAT selective medium is composed of hypoxanthine, aminopterin and thymidine. In this medium, only the hybridoma cells formed by the cell fusion replicate and the myeloma cells die away.

## Table 1

Frequency of occurrence of hybridomas
capable of forming a monoclonal antibody
to aequorin

| Hybridomas | ELISA | | | |
| | Number of wells | Number of hybridomas | Number of aequorin-positive hybridomas | Ratio of the aequorin-positive (%) |
|---|---|---|---|---|
| NZBxNZW, $F_1$ (5 weeks old) | 1728 | 116 | 22 | 19.0 |
| NZBxNZW, $F_1$ (11 weeks old) | 1056 | 27 | 5 | 18.5 |
| BALB/c (5 weeks old) | 708 | 662 | 5 | 0.7 |

Using the antibodies which were found to be aequorin-positive by ELISA, it was determined whether the biochemical activity of aequorin would be neutralized. The results are shown in Table 2.

The number of hybridomas which produced antibody molecules having the ability to inhibit the emission of unique fluorescence by aequorin upon combination with $Ca^{++}$ ions was 6 in the NZBxNZW, $F_1$ mouse group with 5 weeks old, 1 in the 11 week old mouse group, and zero in the BALB/c mouse group.

## Table 2

Antibodies found positive by ELISA, and the number of hybridomas producing antibodies capable of inhibiting emission of luminiscence by aequorin

|  | Hybridoma | ELISA | Inhibition of lumi-nescence |
|---|---|---|---|
| NZBxNZW, $F_1$ (5 weeks old) | 40 | 17 | 6 |
| NZBxNZW, $F_1$ (11 weeks old) | 14 | 5 | 1 |
| BALB/c (5 weeks old) | 500 | 7 | 0 |

## EXAMPLE 2

1) Production of anti-porcine insulin monoclonal antibody:-

NZBxNZW, $F_1$ male mice were sensitized intraperitoneally with 15 micrograms of porcine insulin (a product of Novo) together with Freund's complete adjuvant. 10 to 11 days after the sensitization, 10 micrograms of porcine insulin was administered intraperitoneally together with Freund's incomplete adjuvant. Ten to 21 days after the second sensitization, 3 micrograms of porcine insulin was intravenously administered as final immunization. Three to four days later, the mice were sacrificed, and the spleen was taken. The spleen cells were fused with mouse myeloma cells (NS-1) by the PEG method. The fused cells were distributed in 95-well microtiter plates, and cultivated in HAT medium. Three weeks after the cell fusion, the grown cultivated cells were cloned by the limited dilution method using thymus cells as supporting cells.

2) Selection of an anti-insulin monoclonal antibody:-

The antibody titers were assayed by enzyme-linked

The antibody titers were assayed by enzyme-linked immunosorbent assay (ELISA) on 96-well flat-bottomed microtiter plates. Insulin (derived from bovine, porcine or human) dissolved in a concentration of 1.5 micrograms/ml in sodium carbonate buffer (pH 10) was added at a rate of 100 ml per well, and dried overnight at $37^{O}C$ overnight to fix it to the well. The wells were washed with phosphate-physiological saline buffer. The culture fluid of the hybridoma cells obtained in 1) above was diluted and transferred to the wells and reacted at room temperature over-night or at $37^{O}C$ for 34 hours. The well were thoroughly washed with phosphate-physiological saline buffer, and 100 microliters of a buffer containing 1% goat serum and 0.5 microgram/ml of peroxidase binding rabbit anti-mouse IgG was added to each well. After standing for 2 hours at room temperature, the wells were washed with the above-mentioned buffer. Then, a substrate solution composed of citrate buffer, periodic acid and o-phenylenediamine (a product of Sigma Co.) was added to each well and reacted at room temperature for 30 minutes. The reaction was stopped by sulfuric acid. The absorbance at 492 was measured in each well by EIA reader (MTD-12F2 wavelengths, microplate photometer). Thus, cells which produced antibodies having anti-bovine insulin activity and anti-porcine insulin activity were selected. The resulting monoclonal antibody was designated as JI-1.

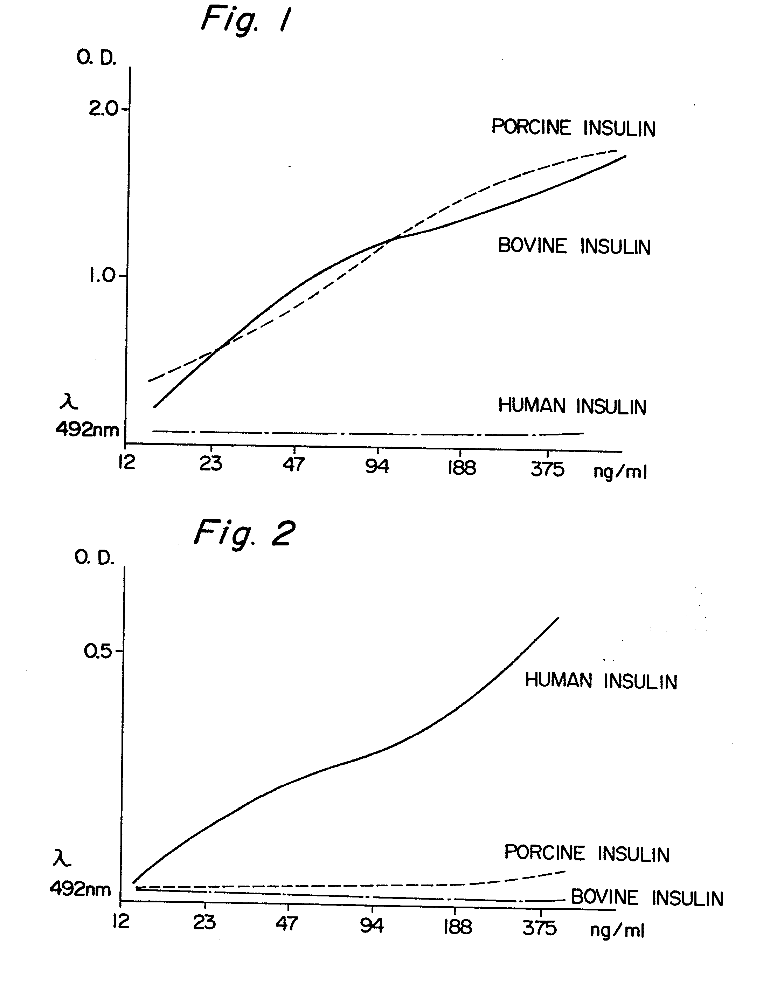

The monoclonal antibody JI-1 was found to belong to IgM as a result of the Ouchterlony's test. JI-1 was subjected to ELISA using insulin derived from porcine, bovine or human as an antigen, and the results are shown in Figure 1.

### EXAMPLE 3

1)      Production of an anti-human insulin monoclonal antibody

NZBxNZW, $F_1$ male mice were sensitized intraperitoneally once with 15 micrograms of human insulin (a product of Novo) together with Freund's complete adjuvant. Ten to 11 days after the sensitization, 10 micrograms of human insulin was intraperioneally administered once together with Freund's incomlete adjuvant. As final immunization, 3 micrograms of human insulin was intravenously injected 10 to 21 days after the second sensitization.

Between the first and the second sensitization, human insulin was once administered subcutaneously, and between the second and the final sensitization, human insulin was subcutaneously administered three times. These insulin administration were effective for the formation of hybridomas.

Three to 4 days after the final immunization, the mice were sacrificed, and the spleen was taken. The spleen cells were fused with mouse myeloma cells (NS-1) by the PEG method. The fused cells were distributed on 96-well microtiter plates and cultivated. Three weeks after the cell fusion, the cultivated cells were cloned by the limited dilution method.

2)        Selection of anti-insulin monoclonal antibody:-
By the same way as in Example 3, anti-human insulin monoclonal antibody-producing cells were obtained from many monoclonal antibody-producing cells. The resulting monoclonal antibodies were designated as JI-2. They were found to belong to IgG as a result of the Ouchterlony's test.

These monoclonal antibodies JI-2 were subjected to ELISA using insulin derived from porcine, bovine or human as an antigen. The results are shown in Figure 2.

EXAMPLE 4

Comparison of monoclonal antibody-producing hybridomas by mouse species:-
Twenty-four BALB/c mice were divided into two groups and immunized with human insulin and porcine insulin, respectively, and 1238 hybridomas and 3773 hybridomas were obtained, respectively. Out of these, 135 hybridomas produced anti-human insulin antibodies, and 410 hybridomas produced anti-porcine insulin antibodies.

Then, 34 NZBxNZW, $F_1$ mice were divided into three groups, and immunized respectively with human insulin, porcine insulin and bovine insulin, and 1380 hybridomas, 1281 hybridomas and 624 hybridomas were respectively obtained. Out of these, 270 hybridomas produced anti-human insulin antibodies; 251 hybridomas, anti-porcine insulin antibodies; and 122 hybridomas, anti-bovine insulin antibodies.

Immunizations with insulin and tests for the reactivity of the antibodies with various insulins were carried out in in accordance with the method of Example 1 or 2. The results of the test are shown in Table 3.

Table 3

| Mouse | Immunizing source \ Antigen | Human insulin | Porcine insulin | Bovine insulin | Human and porcine insulins | Human and bovine insulins | Porcine and bovine insulins | Human, porcine and bovine insulins |
|---|---|---|---|---|---|---|---|---|
| BALB/c | Human insulin | 18 | 7 | 5 | 2 | 3 | 4 | 96 |
|  | Porcine insulin | 31 | 15 | 25 | 23 | 15 | 10 | 291 |
| NZBxNZW, $F_1$ | Human insulin | 5 | 9 | 6 | 7 | 4 | 1 | 238 |
|  | Porcine insulin | 12 | 1 | 6 | 5 | 4 | 2 | 221 |
|  | Bovine insulin | 11 | 0 | 0 | 2 | 2 | 0 | 107 |

EXAMPLE 5

Production of cells capable of producing
a monoclonal antibody to insulin:-

Insulin is a proteinous hormone having a relatively low molecular weight, and it is known that human insulin is very similar to porcine insulin.

NZBxNZW, $F_1$ mice (4 weeks old) were immunized with both human and porcine insulins by injecting each insulin in an amount of 50 micrograms for the first time, 25 micrograms for the second time and 3 micrograms finally. Three days after the final immunization, spleen cells as a suspension prepared from the spleen of the mice by pressing the tissues through a stainless steel mesh were fused with mouse myeloma cells by the PEG method in HAT selective medium.

The specificities of the resulting hybridoma cells to the antigens were examined, and the results are shown in Table 4.

When NZBxNZW, $F_1$ were immunized with human insulin as an antigen, 220 hybridomas were obtained. The number of hybridomas which were found to be positive by ELISA was 32 (14.5%). When porcine insulin was used as the antigen, 200 hybridomas were obtained. Sixty-four out of these showed specificity to the antigen, and the ratio of hybridomas which were found to be positive by ELISA was 32.0%.

Table 4

Frequency of occurrence of hybridomas capable of
producing a monoclonal antibody to insulin

| Antigen | Number of hybridomas | | |
|---|---|---|---|
| | Total | Anti-antigen positive | Ratio of positive hybridomas (%) |
| Human insulin | 220 | 32 | 14.5 |
| Porcine insulin | 200 | 64 | 32.0 |

EXAMPLE 6

Purification of human monocomponent insulin
(semi-synthesized enzymatically by modification
of porcine insulin)

1)        Preparation of monoclonal antibody JI-2 column:-

1 g of CNBr-activated Sepharose 4B (Pharmacia) was coupled with 15 mg of monoclonal antibody JI-2 obtained in Example 3 in a conventional manner to give monoclonal antibody JI-2 column.

2)        Purification of human monocomponent insulin:-

1 mg of human monocomponent insulin dissolved in 10 ml of 0.01 M Tris hydrochloric acid buffer solution containing 0.15 M NaCl, pH 8.0, was applied to the antibody-column obtained in item 1) above at a flow rate of 0.5 ml per hour and then washed with 50 ml of the same buffer solution as described above. Subsequently human type insulin was eluted out with 10 ml of 1 M acetic acid solution and then lyophilized to give 0.8 g of immunologically pure human monocomponent insulin.

- 17 -

## CLAIMS

1. A monoclonal antibody having specificity to an immunologically tolerant hormone or enzyme.

2. A monoclonal antibody according to claim 1 from hybridoma cells derived by fusion of antibody producing cells from an $F_1$ mouse of NZBxNZW strain, an NZB strain mouse, a BxSB strain mouse, an MRL/1 strain mouse, or an MRL/n strain mouse with myeloma cells.

3. A monoclonal antibody according to claim 2 wherein the myeloma cells are mouse myeloma cells.

4. A monoclonal antibody according to claim 1, 2 or 3 wherein the immunologically tolerant hormone is insulin.

5. A monoclonal antibody according to claim 1, 2 or 3 wherein the immunologically tolerant enzyme is aequorin.

6. A process for producing cells capable of producing a monoclonal antibody as claimed in any one of claims 1 to 5, which comprises immunizing an animal in a state of autoimmune disease or hereditarily susceptible to autoimmune disease with a hormone or enzyme which is immunologically tolerant to a normal animal of the same species as said animal, fusing the resulting antibody-producing cells with myeloma cells, isolating the antibody-producing hybridoma cells thus obtained, and cloning the hybridoma cells.

7. A method for the purification of an immunologically tolerant hormone or enzyme which comprises contacting an impure such hormone or enzyme with a monoclonal antibody as claimed in any one of claims 1 to 5.

8. A method according to claim 7 wherein the hormone is an insulin.

9. A method according to claim 8 wherein the insulin is humanized porcine-derived insulin.

*Fig. 1*

*Fig. 2*